# EUROPEAN PATENT APPLICATION

(11) **EP 4 785 924 A1**
(43) Date of publication of application: **05.08.2026**
(21) Application number: 24888894.3
(22) Date of filing: 16.08.2024
(51) Int. Cl.: A61K 8/06, A61K 8/893, A61K 8/891, A61K 8/88, A61K 8/34, A61K 8/67, A61K 8/60, A61K 8/42, A61K 8/9789

(54) **WATER-IN-OIL TYPE NANOEMULSION COMPOSITION, METHOD FOR PREPARING SAME, AND COSMETIC MATERIAL COMPOSITION COMPRISING SAME**

(30) Priority: 07.11.2023 KR 20230153025; 05.08.2024 KR 20240104197
(71) Applicant: Cosmax, Inc., Hwaseong-si, Gyeonggi-do 18622 (KR)
(72) Inventor: SEO, Jungwon, Seongnam-si, Gyeonggi-do 13486 (KR); EOM, Sewon, Seongnam-si, Gyeonggi-do 13486 (KR); AHN, Yehee, Seongnam-si, Gyeonggi-do 13486 (KR); AHN, Youjin, Seongnam-si, Gyeonggi-do 13486 (KR); LEE, Whayoung, Seongnam-si, Gyeonggi-do 13486 (KR); PARK, Cheon Ho, Seongnam-si, Gyeonggi-do 13486 (KR)
(74) Representative: Mammel und Maser Patentanwälte PartG mbB
(86) International application number: PCT/KR2024/012195
(87) International publication number: WO 2025/100698

(57) **Abstract**

The present invention relates to a water-in-oil type nanoemulsion composition comprising a surfactant, a skin conditioning agent, and a polyol. The nanoemulsion composition according to the present invention has excellent moisturizing properties, feeling of use, and skin absorption. In addition, the present invention provides a water-in-oil type UV nanoemulsion cosmetic material composition which is for UV protection and makeup and has excellent UV blocking ability and makeup effects. In addition, the present invention provides a method for preparing a water-in-oil type nanoemulsion composition having excellent formulation stability.

## Description

### [Technical Field]

The present disclosure relates to a water-in-oil nanoemulsion composition, a preparation method therefor, and a cosmetic composition comprising same and, more particularly, to a water-in-oil nanoemulsion composition applicable to sunscreen and makeup cosmetic compositions and a preparation method therefor.

### [Background Art]

With reduced particle sizes, nanoemulsions improve skin absorption of cosmetic compositions, provide excellent moisturizing effects, and reduce stickiness, thereby offering superior usability. Accordingly, conventional nanoemulsion cosmetic compositions have been mainly developed and commercialized as basic cosmetic products, such as toners, ampoules, and creams, to which oil-in-water nanoemulsions are applied.

However, oil-in-water emulsions have disadvantages in that makeup effects such as coverage, adhesion, and durability are poor, and thus are not suitable for makeup cosmetic compositions such as foundations.

Low-viscosity cosmetic compositions have advantages such as a light texture, fast adhesion, excellent spreadability, and uniform application. However, as viscosity decreases, phenomena such as creaming, flocculation, coalescence, and Ostwald ripening may occur, resulting in low stability.

Accordingly, there is a need for development of a makeup cosmetic composition that has the advantages of nanoemulsions, such as excellent moisturizing properties and usability, while also having high coverage, excellent adhesion, and long-lasting effects, and exhibiting high stability even at low viscosity.

### [Disclosure]

### [Technical Problem]

In order to solve the problems encountered in the related art as described above, the present disclosure aims to provide a water-in-oil nanoemulsion composition.

In addition, the present disclosure aims to provide a method for preparing a water-in-oil nanoemulsion composition.

Also, the present disclosure aims to provide a water-in-oil sunscreen and makeup nanoemulsion cosmetic composition comprising the water-in-oil nanoemulsion composition.

### [Technical Solution]

One aspect of the present disclosure provides a method for preparing a water-in-oil nanoemulsion composition. The method for preparing the water-in-oil nanoemulsion composition may include the steps of: preparing an oil phase including a surfactant and a skin conditioning agent (S1); preparing an aqueous phase including a polyol (S2); mixing the oil phase and the aqueous phase (S3); and emulsifying the mixture of step (S3) using a microfluidizer (S4).

The surfactant may include one or more selected from the group consisting of PEG-10 dimethicone, cetyl PEG/PPG-10/1 dimethicone, lauryl PEG-9 polydimethylsiloxyethyl dimethicone, lauryl PEG-10 tris(trimethylsiloxy)silyl ethyl dimethicone, PEG-9 polydimethylsiloxyethyl dimethicone, bis-PEG/PPG-14/14 dimethicone, bis-PEG/PPG-20/20 dimethicone, PEG/PPG-18/18 dimethicone, PEG/PPG-19/19 dimethicone, PEG/PPG-20/20 dimethicone, PEG-8 dimethicone, bis-PEG-12 dimethicone, lauryl PEG-8 dimethicone, polyglyceryl-3 polydimethylsiloxyethyl dimethicone, lauryl polyglyceryl-3 polydimethylsiloxyethyl dimethicone, dimethicone/PEG-10/15 crosspolymer, dimethicone/polyglycerin-3 crosspolymer, lauryl dimethicone/polyglycerin-3 crosspolymer, sorbitan stearate, sorbitan oleate, sorbitan isostearate, sorbitan sesquioleate, PEG-30 dipolyhydroxystearate, polyglyceryl-2 isostearate, polyglyceryl-2 diisostearate, polyglyceryl-2 tetraisostearate, polyglyceryl-2 oleate, polyglyceryl-2 dipolyhydroxystearate, polyglyceryl-3 distearate, polyglyceryl-3 diisostearate, polyglyceryl-3 polyricinoleate, polyglyceryl-4 isostearate, polyglyceryl-4 oleate, polyglyceryl-4 tristearate, polyglyceryl-4 pentastearate, polyglyceryl-4 pentaoleate, polyglyceryl-4 polyricinoleate, polyglyceryl-4 diisostearate/polyhydroxystearate/sebacate, polyhydroxystearic acid, polyglyceryl-6 pentastearate, polyglyceryl-6 pentaoleate, polyglyceryl-6 polyricinoleate, polyglyceryl-10 decaethylhexanoate, polyglyceryl-10 decastearate, polyglyceryl-10 decaisostearate, polyglyceryl-10 pentaisostearate, polyglyceryl-10 decaoleate, polyglyceryl-10 decaoleate, and polyglyceryl-10 dodecabehenate. In addition, the surfactant may be contained in an amount of 10 to 20 wt% based on the total weight of the composition.

The skin conditioning agent may include one or more selected from the group consisting of hydrocarbon oils, ester oils, silicone oils, polyesters, fatty acids and/or fatty alcohols, animal oils, vegetable oils, mineral oils, and mixtures thereof, and may further include one or more selected from the group consisting of disiloxane, trisiloxane, dimethicone, methyl trimethicone, cyclopentasiloxane, cyclohexasiloxane, caprylyl methicone, cetyl dimethicone, phenyl trimethicone, diphenylsiloxy phenyl trimethicone, diphenyl dimethicone, trimethylsiloxy phenyl dimethicone, trimethylsiloxysilicate, phenylpropyl dimethylsiloxysilicate, acrylates/dimethicone copolymer, acrylates/polytrimethylsiloxymethacrylate copolymer, acrylates/stearyl acrylate/dimethicone methacrylate copolymer, polypropylsilsesquioxane, polymethylsilsesquioxane, polyphenylsilsesquioxane, vinyl dimethicone/methicone silsesquioxane crosspolymer, dimethicone/vinyl dimethicone crosspolymer, dimethicone crosspolymer, dimethicone/PEG-10/15 crosspolymer, dimethicone/polyglycerin-3 crosspolymer, lauryl dimethicone/polyglycerin-3 crosspolymer, dilinoleic acid/butanediol copolymer, castor oil/IPDI copolymer, coco-caprylate/caprate, triheptanoin, dipropylene glycol dibenzoate, octyldodecanol, tridecyl trimellitate, caprylic/capric triglyceride, decyl cocoate, C12-15 alkyl benzoate, isononyl isononanoate, cetyl ethylhexanoate, butylene glycol dicaprylate/dicaprate, isododecane, C15-19 alkane, C9-12 alkane, squalane, and hydrogenated coconut oil. In addition, the skin conditioning agent may be contained in an amount of 20 to 50 wt% based on the total weight of the composition.

The surfactant and the skin conditioning agent may be contained in a sum amount of 30 to 70 wt% based on the total weight of the composition. In addition, a weight ratio of the surfactant to the skin conditioning agent may be 1:1 to 1:5.

The polyol may include one or more selected from the group consisting of propanediol, propylene glycol, methylpropanediol, dipropylene glycol, butylene glycol, diglycerin, glycerin, pentylene glycol, and 1,2-hexanediol. In addition, the polyol may be contained in an amount of 0.1 to 50 wt% based on the total weight of the composition.

The step (S4) may be performed under a pressure of 100 to 1500 bar and at a temperature of 0 to 70°C. In addition, the step (S4) may be performed one or more times.

Another aspect of the present disclosure provides a water-in-oil nanoemulsion composition including a surfactant, a skin conditioning agent, and a polyol.

Another aspect of the present disclosure provides a water-in-oil sunscreen and makeup nanoemulsion cosmetic composition comprising the water-in-oil nanoemulsion composition.

The water-in-oil nanoemulsion composition may be contained in an amount of 1 to 99 wt% based on the total weight of the water-in-oil sunscreen and makeup nanoemulsion cosmetic composition.

The water-in-oil sunscreen and makeup nanoemulsion cosmetic composition may further comprise a powder. The powder may comprise one or more selected from the group consisting of titanium dioxide, fine particle titanium dioxide, zinc oxide, yellow iron oxide, red iron oxide, black iron oxide, ultramarine, and chromium oxide green. In addition, the powder may be contained in an amount of 0.1 to 50 wt% based on the total weight of the composition.

### [Advantageous Effects]

Provided according to one embodiment of the present disclosure may be a water-in-oil sunscreen and makeup nanoemulsion cosmetic composition having improved moisturizing properties, usability, and skin absorption.

In addition, provided according to one embodiment of the present disclosure may be a water-in-oil sunscreen and makeup nanoemulsion cosmetic composition having high coverage, excellent adhesion, and long-lasting effects.

Furthermore, provided according to one embodiment of the present disclosure may be a water-in-oil sunscreen and makeup nanoemulsion cosmetic composition having high stability even at low viscosity.

### [Description of Drawings]

FIG. 1 is a photographic image showing the results of a transparency test according to one embodiment of the present disclosure.
FIG. 2 shows photographic images illustrating the results of a formulation stability test according to one embodiment of the present disclosure.
FIG. 3 is a plot illustrating the results of a moisture retention test according to one embodiment of the present disclosure.
FIGS. 4 to 8 are photographic images illustrating the results of formulation stability tests according to other embodiments of the present disclosure.

### [Best Mode for Invention]

Below, a detailed description will be given of the present disclosure with reference to Examples. However, these Examples are set forth for illustrative purposes, but are not to be construed to limit the scope of the present disclosure.

According to one aspect of the present disclosure, a method for preparing a water-in-oil nanoemulsion composition is provided.

The preparation method may include the steps of: preparing an oil phase including a surfactant and a skin conditioning agent (S1); preparing an aqueous phase including a polyol (S2); mixing the oil phase and the aqueous phase (S3); and emulsifying the mixture of step (S3) using a microfluidizer (S4).

The emulsifying step (S4) using the microfluidizer may be performed at 100 to 1500 bar. In the present disclosure, the step (S4) may be performed at 100 bar or more, 200 bar or more, 300 bar or more, 400 bar or more, or 500 bar or more, and at 1500 bar or less, 1300 bar or less, 1100 bar or less, 900 bar or less, or 700 bar or less. When the step (S4) is performed at less than 100 bar, there is a problem in that the force for pushing the mixture is insufficient, so that the mixture cannot pass through the apparatus or a nanoemulsion cannot be formed. When the step (S4) is performed at more than 1500 bar, phase separation may occur, thereby preventing formation of a nanoemulsion.

The emulsifying step (S4) using the microfluidizer may be performed at 0 to 70°C. In the present disclosure, the step (S4) may be performed at 0°C or more, 10°C or more, 20°C or more, 30°C or more, 40°C or more, 50°C or more, or 60°C or more, and at 70°C or less, 60°C or less, 50°C or less, 40°C or less, or 30°C or less. When the step (S4) is performed at less than 0°C, there are limitations in terms of equipment, and when it is performed at more than 70°C, phase separation may occur, thereby preventing formation of a nanoemulsion.

The emulsifying step (S4) using the microfluidizer may be performed once or two or more times. In the present disclosure, when the step (S4) is performed two or more times, the average particle diameter of the aqueous phase may become smaller and more uniform.

According to another aspect of the present disclosure, a water-in-oil nanoemulsion composition including a surfactant, a skin conditioning agent, and a polyol is provided.

The water-in-oil nanoemulsion composition may further include an active ingredient, purified water, and other additives.

The water-in-oil nanoemulsion composition of the present disclosure may have an average particle diameter of the aqueous phase of 10 to 1000 nm. In the present disclosure, the average particle diameter of the aqueous phase may be 10 nm or more, 50 nm or more, 100 nm or more, 150 nm or more, 200 nm or more, or 250 nm or more, and may be 1000 nm or less, 900 nm or less, 800 nm or less, 700 nm or less, 600 nm or less, or 500 nm or less.

The water-in-oil nanoemulsion composition of the present disclosure may be prepared by the method for preparing a water-in-oil nanoemulsion composition of the present disclosure.

According to another aspect of the present disclosure, a water-in-oil sunscreen and makeup nanoemulsion cosmetic composition comprising the water-in-oil nanoemulsion composition is provided.

The water-in-oil nanoemulsion composition may be contained an amount of 1 to 99 wt% based on the total weight of the cosmetic composition. In the present disclosure, the water-in-oil nanoemulsion composition may be contained in an amount of 1 wt% or more, 10 wt% or more, 20 wt% or more, 30 wt% or more, 40 wt% or more, or 50 wt% or more, and may be included in an amount of 99 wt% or less, 90 wt% or less, 80 wt% or less, 70 wt% or less, or 60 wt% or less.

The water-in-oil sunscreen and makeup nanoemulsion cosmetic composition may further include a powder.

The water-in-oil sunscreen and makeup nanoemulsion cosmetic composition may have a viscosity of 100 to 20,000 cps. In the present disclosure, the viscosity refers to a value measured using a Brookfield viscometer under conditions of spindle No. 4, 12 rpm, 1 minute, and 25°C. In the present disclosure, the viscosity of the cosmetic composition may be 100 cps or more, 200 cps or more, 500 cps or more, 800 cps or more, 1,000 cps or more, 1,500 cps or more, or 2,000 cps or more, and may be 20,000 cps or less, 18,000 cps or less, 15,000 cps or less, 12,000 cps or less, 10,000 cps or less, 9,000 cps or less, 8,000 cps or less, 7,000 cps or less, or 6,000 cps or less.

### A. Surfactant

The composition of the present disclosure includes a surfactant. In the present disclosure, the surfactant affects the stability of the emulsion formulation.

The surfactant may be one or more selected from the group consisting of PEG-10 dimethicone, cetyl PEG/PPG-10/1 dimethicone, lauryl PEG-9 polydimethylsiloxyethyl dimethicone, lauryl PEG-10 tris(trimethylsiloxy)silyl ethyl dimethicone, PEG-9 polydimethylsiloxyethyl dimethicone, bis-PEG/PPG-14/14 dimethicone, bis-PEG/PPG-20/20 dimethicone, PEG/PPG-18/18 dimethicone, PEG/PPG-19/19 dimethicone, PEG/PPG-20/20 dimethicone, PEG-8 dimethicone, bis-PEG-12 dimethicone, lauryl PEG-8 dimethicone, polyglyceryl-3 polydimethylsiloxyethyl dimethicone, lauryl polyglyceryl-3 polydimethylsiloxyethyl dimethicone, dimethicone/PEG-10/15 crosspolymer, dimethicone/polyglycerin-3 crosspolymer, lauryl dimethicone/polyglycerin-3 crosspolymer, sorbitan stearate, sorbitan oleate, sorbitan isostearate, sorbitan sesquioleate, PEG-30 dipolyhydroxystearate, polyglyceryl-2 isostearate, polyglyceryl-2 diisostearate, polyglyceryl-2 tetraisostearate, polyglyceryl-2 oleate, polyglyceryl-2 dipolyhydroxystearate, polyglyceryl-3 distearate, polyglyceryl-3 diisostearate, polyglyceryl-3 polyricinoleate, polyglyceryl-4 isostearate, polyglyceryl-4 oleate, polyglyceryl-4 tristearate, polyglyceryl-4 pentastearate, polyglyceryl-4 pentaoleate, polyglyceryl-4 polyricinoleate, polyglyceryl-4 diisostearate/polyhydroxystearate/sebacate, polyhydroxystearic acid, polyglyceryl-6 pentastearate, polyglyceryl-6 pentaoleate, polyglyceryl-6 polyricinoleate, polyglyceryl-10 decaethylhexanoate, polyglyceryl-10 decastearate, polyglyceryl-10 decaisostearate, polyglyceryl-10 pentaisostearate, polyglyceryl-10 decaoleate, polyglyceryl-10 decaoleate, and polyglyceryl-10 dodecabehenate.

The surfactant may be contained in an amount of 10 to 20 wt% based on the total weight of the composition. In the present disclosure, the surfactant may be contained in an amount of 10 wt% or more, 11 wt% or more, 12 wt% or more, 13 wt% or more, or 14 wt% or more, and may be contained in an amount of 20 wt% or less, 19 wt% or less, 18 wt% or less, 17 wt% or less, 16 wt% or less, or 15 wt% or less. When the surfactant is contained in an amount of less than 10 wt%, a nanoemulsion may not be formed, and when it is contained in an amount exceeding 20 wt%, there is a problem in that the stability of the formulation is reduced.

### B. Skin Conditioning Agent

The composition of the present disclosure comprises a skin conditioning agent. In the present disclosure, the skin conditioning agent may improve moisture retention and provide a soft feel during use.

The skin conditioning agent may comprise one or more selected from the group consisting of hydrocarbon oils, ester oils, silicone oils, polyesters, fatty acids and/or fatty alcohols, animal oils, vegetable oils, mineral oils, and mixtures thereof. For example, the skin conditioning agent may comprise one or more selected from the group consisting of disiloxane, trisiloxane, dimethicone, methyl trimethicone, cyclopentasiloxane, cyclohexasiloxane, caprylyl methicone, cetyl dimethicone, phenyl trimethicone, diphenylsiloxy phenyl trimethicone, diphenyl dimethicone, trimethylsiloxy phenyl dimethicone, trimethylsiloxysilicate, phenylpropyl dimethylsiloxysilicate, acrylates/dimethicone copolymer, acrylates/polytrimethylsiloxymethacrylate copolymer, acrylates/stearyl acrylate/dimethicone methacrylate copolymer, polypropylsilsesquioxane, polymethylsilsesquioxane, polyphenylsilsesquioxane, vinyl dimethicone/methicone silsesquioxane crosspolymer, dimethicone/vinyl dimethicone crosspolymer, dimethicone crosspolymer, dimethicone/PEG-10/15 crosspolymer, dimethicone/polyglycerin-3 crosspolymer, lauryl dimethicone/polyglycerin-3 crosspolymer, dilinoleic acid/butanediol copolymer, castor oil/IPDI copolymer, coco-caprylate/caprate, triheptanoin, dipropylene glycol dibenzoate, octyldodecanol, tridecyl trimellitate, caprylic/capric triglyceride, decyl cocoate, C12-15 alkyl benzoate, isononyl isononanoate, cetyl ethylhexanoate, butylene glycol dicaprylate/dicaprate, isododecane, C15-19 alkane, C9-12 alkane, squalane, and hydrogenated coconut oil.

The skin conditioning agent may be contained in an amount of 20 to 50 wt% based on the total weight of the composition. In the present disclosure, the skin conditioning agent may be contained in an amount of 20 wt% or more, 22 wt% or more, 25 wt% or more, 28 wt% or more, or 30 wt% or more, and may be contained in an amount of 50 wt% or less, 45 wt% or less, 40 wt% or less, 35 wt% or less, or 30 wt% or less. When the skin conditioning agent is included in an amount of less than 20 wt%, a nanoemulsion may not be formed and phase separation may occur, and when it is contained in an amount exceeding 50 wt%, the proportion of the external phase (oil phase) increases, thereby reducing the advantages of the nanoemulsion.

In addition, the total amount of the surfactant and the skin conditioning agent may be contained in an amount of 30 to 70 wt% based on the total weight of the composition. In the present disclosure, the total amount of the surfactant and the skin conditioning agent may be contained in an amount of 30 wt% or more, 35 wt% or more, or 40 wt% or more, and may be contained in an amount of 70 wt% or less, 65 wt% or less, 60 wt% or less, 55 wt% or less, or 50 wt% or less.

The weight ratio of the surfactant to the skin conditioning agent may be 1:1 to 1:5. In the present disclosure, the weight ratio of the surfactant to the skin conditioning agent may be 1:1 to 1:5, 1:1 to 1:4, 1:1 to 1:3.5, or 1:1 to 1:3.

### C. Polyol

The composition of the present disclosure comprises a polyol. In the present disclosure, the polyol may improve moisture retention and enhance formulation stability.

The polyol may be one or more selected from the group consisting of propanediol, propylene glycol, methylpropanediol, dipropylene glycol, butylene glycol, diglycerin, glycerin, pentylene glycol, and 1,2-hexanediol.

The polyol may be contained in an amount of 0.1 to 50 wt% based on the total weight of the composition. In the present disclosure, the polyol may be contained in an amount of 0.1 wt% or more, 5 wt% or more, 10 wt% or more, 15 wt% or more, or 20 wt% or more, and may be included in an amount of 50 wt% or less, 45 wt% or less, 40 wt% or less, 35 wt% or less, or 30 wt% or less. When the polyol is contained in an amount of less than 0.1 wt%, the viscosity is excessively increased, resulting in difficulty in passing through a microfluidizer, and when it is contained in an amount exceeding 50 wt%, emulsification may not occur and phase separation may occur.

### D. Active Ingredient

The composition of the present disclosure may include an active ingredient.

In the present disclosure, the active ingredient may be any water-soluble physiologically active component known in the art, and for example, may be one or more selected from the group consisting of niacinamide, adenosine, panthenol, Centella asiatica extract, hyaluronic acid, ceramide NP, caffeine, madecassoside, madecassic acid, arbutin, cholesterol, amino acids, vitamins, collagen, and proteins.

The active ingredient may be contained in an amount of 0.001 to 20 wt% based on the total weight of the composition. In the present disclosure, the active ingredient may be contained in an amount of 0.001 wt% or more, 0.005 wt% or more, 0.01 wt% or more, 0.05 wt% or more, 0.1 wt% or more, 0.5 wt% or more, 1 wt% or more, or 2 wt% or more, and may be included in an amount of 20 wt% or less, 15 wt% or less, 10 wt% or less, 7 wt% or less, or 5 wt% or less. When the active ingredient is contained in an amount of less than 0.001 wt%, the efficacy of the active ingredient may not be sufficiently exhibited, and when it is contained in an amount exceeding 20 wt%, the stability of the formulation may be reduced.

### E. Powder

The composition of the present disclosure may comprise a powder. In the present disclosure, the powder may be dispersed in the oil phase.

In the present disclosure, the powder may be any powder known in the art, and for example, may be one or more selected from the group consisting of titanium dioxide, fine titanium dioxide, zinc oxide, yellow iron oxide, red iron oxide, black iron oxide, ultramarine, and chromium oxide green.

The powder may be contained in an amount of 0.1 to 50 wt% based on the total weight of the composition. In the present disclosure, the powder may be contained in an amount of 0.1 wt% or more, 5 wt% or more, 10 wt% or more, 15 wt% or more, or 20 wt% or more, and may be contained in an amount of 50 wt% or less, 45 wt% or less, 40 wt% or less, 35 wt% or less, or 30 wt% or less. When the powder is contained in an amount of less than 0.1 wt%, there is a problem in that makeup and ultraviolet-blocking functions such as water resistance, spreadability, usability, durability, and coverage cannot be achieved, and when it is contained in an amount exceeding 50 wt%, the stability of the formulation is reduced.

### F. Purified Water

The composition of the present disclosure may comprise purified water. In the present disclosure, the content of the purified water may be a balance sufficient to satisfy 100% of the composition, and for example, may be contained in an amount of 1 to 65 wt% based on the total weight of the composition.

### G. Other Additives

Meanwhile, the composition of the present disclosure may further comprise additives commonly used in the art to which the present disclosure pertains, depending on the intended use, within a range that does not impair the purpose and effects of the present disclosure. Preferably, at least one selected from the group consisting of humectants, metal ion sequestrants, emollients, thickeners, preservatives, and stabilizers may be included, but the present disclosure is not limited thereto.

In the present disclosure, the above-described other additives may be contained in an amount of 0.1 to 10 wt%.

### [Mode for Invention]

Hereinafter, preferred examples are described in order to describe the present disclosure in more detail. However, the following examples are provided only for illustrating the present disclosure and are not intended to limit the present disclosure.

### <Preparation Example 1 of Nanoemulsion Composition>

Nanoemulsion compositions having different contents of a surfactant were prepared according to the compositions (wt%) shown in Table 1 below.

**[TABLE 1]**

| | Component(wt%) | C. Ex. 1 | C. Ex. 2 | C. Ex. 3 | C. Ex. 4 | C. Ex. 5 | C. Ex. 6 |
|---|---|---|---|---|---|---|---|
| Oil phase | PEG-10 dimethicone | 0.5 | 5 | 6 | 7 | 8 | 9 |
| | Dimethicone | 30 | 30 | 30 | 30 | 30 | 30 |
| Water phase | Purified water | Balance | Balance | Balance | Balance | Balance | Balance |
| | Glycerin | 10 | 10 | 10 | 10 | 10 | 10 |
| | Propanediol | 5 | 5 | 5 | 5 | 5 | 5 |
| | Other additives | 5 | 5 | 5 | 5 | 5 | 5 |
| Sum | | 100.0 | 100.0 | 100.0 | 100.0 | 100.0 | 100.0 |

| | Component(wt%) | Ex. 1 | Ex. 2 | Ex. 3 | Ex. 4 | Ex. 5 | Ex. 6 |
|---|---|---|---|---|---|---|---|
| Oil phase | PEG-10 dimethicone | 10 | 11 | 15 | 20 | 21 | 25 |
| | Dimethicone | 30 | 30 | 30 | 30 | 30 | 30 |
| Water phase | Purified water | Balance | Balance | Balance | Balance | Balance | Balance |
| | Glycerin | 10 | 10 | 10 | 10 | 10 | 10 |
| | Propanediol | 5 | 5 | 5 | 5 | 5 | 5 |
| | Other additives | 5 | 5 | 5 | 5 | 5 | 5 |
| Sum | | 100.0 | 100.0 | 100.0 | 100.0 | 100.0 | 100.0 |

Specifically, after weighing according to the composition of Table 1, an oil phase part and an aqueous phase part were each heated and dissolved at 30°C, and the aqueous phase part was gradually added to the oil phase part while stirring at 3000 rpm for 10 minutes using a homogenizer to prepare a conventional emulsion. The conventional emulsion was then subjected to an emulsification process twice using a microfluidizer at a temperature of 30°C and a pressure of 500 bar to prepare oil-in-water nanoemulsion compositions of Comparative Examples 1 to 6 and Examples 1 to 6.

### Formulation Implementation Test

A formulation implementation test was performed on the compositions of the Examples and Comparative Examples.

Each composition prepared in the Examples and Comparative Examples was visually observed, and the particle size of the composition diluted in oil was measured by static light scattering (SLS) using an LA-960 particle size analyzer (Horiba, Japan). The results of the evaluation of formulation appearance and the average particle size are shown in Table 2 below.

### - Criteria for formulation evaluation

⊚: very good, ○: good, △: fair, ×: phase separation

**[TABLE 2]**

| | C. Ex. 1 | C. Ex. 2 | C. Ex. 3 | C. Ex. 4 | C. Ex. 5 | C. Ex. 6 |
|---|---|---|---|---|---|---|
| Evaluation for formulation implementation | × | × | × | × | × | × |
| Avg. diameter(nm) | - | - | - | - | - | - |

| | Ex. 1 | Ex. 2 | Ex. 3 | Ex. 4 | Ex. 5 | Ex. 6 |
|---|---|---|---|---|---|---|
| Evaluation for formulation implementation | ⊚ | ⊚ | ⊚ | ⊚ | ⊚ | ⊚ |
| Avg. diameter(nm) | 250 | 250 | 240 | 230 | 230 | 230 |

As shown in Table 2, it was confirmed that in Examples 1 to 6, nanoemulsion compositions having an average particle size of 230 to 250 nm were successfully implemented. However, in Comparative Examples 1 to 6 having a low content of the surfactant, the emulsion was not formed after passing through the microfluidizer and phase separation was observed. Meanwhile, Examples 5 and 6, which had a relatively high content of the surfactant, were evaluated as having poor absorbability and high stickiness, and thus were found to be unsuitable for application to cosmetic compositions.

### <Preparation Example 2 of Nanoemulsion Composition>

General emulsion and nanoemulsion compositions having different contents of a surfactant were prepared according to the compositions (wt%) shown in Table 3 below.

**[TABLE 3]**

| | Component(wt%) | C. Ex. 7 | C. Ex. 8 | C. Ex. 9 | C. Ex. 10 | C. Ex. 11 | C. Ex. 7 |
|---|---|---|---|---|---|---|---|
| Oil phase | PEG-10 dimethicone | 5 | 7 | 10 | 5 | 7 | 10 |
| | Dimethicone | 20 | 20 | 20 | 20 | 20 | 20 |
| Water phase | Purified water | Balance | Balance | Balance | Balance | Balance | Balance |
| | Glycerin | 10 | 10 | 10 | 10 | 10 | 10 |
| | Propanediol | 5 | 5 | 5 | 5 | 5 | 5 |
| | Other additives | 5 | 5 | 5 | 5 | 5 | 5 |
| Sum | | 100.0 | 100.0 | 100.0 | 100.0 | 100.0 | 100.0 |

In Comparative Examples 7 to 9, an oil phase part and an aqueous phase part were each heated and dissolved at 30°C, and the aqueous phase part was gradually added to the oil phase part while stirring at 3000 rpm for 10 minutes using a homogenizer to prepare water-in-oil general emulsion compositions. Comparative Examples 10 and 11 and Example 7 were prepared as water-in-oil nanoemulsion compositions in the same manner as in Example 1.

### Formulation Implementation Test

A formulation implementation test was performed on the compositions of the Examples and Comparative Examples.

Each composition prepared in the Examples and Comparative Examples was visually observed, and the particle size of the composition diluted in oil was measured by static light scattering (SLS) using an LA-960 particle size analyzer (Horiba, Japan). The results of the evaluation of formulation appearance and the average particle size are shown in Table 4 below.

### - Criteria for formulation evaluation

⊚: very good, ○: good, △: fair, ×: phase separation

**[TABLE 4]**

| | C. Ex. 7 | C. Ex. 8 | C. Ex. 9 | C. Ex. 10 | C. Ex. 11 | C. Ex. 7 |
|---|---|---|---|---|---|---|
| Evaluation for formulation implementation | ○ | ○ | ○ | × | × | ⊚ |
| Avg. diameter(nm) | 4472 | 5122 | 7690 | - | - | 260 |

As shown in Table 4, it was confirmed that in Example 7, a nanoemulsion composition having an average particle size of 260 nm was successfully implemented. However, in Comparative Examples 10 and 11 having a low content of the surfactant, phase separation was observed, and in Comparative Examples 7 to 9, it was confirmed that the average particle size was large, ranging from 4000 to 8000 nm. Comparative Examples 10 and 11 included the same content of the surfactant as Comparative Examples 7 and 8, respectively; however, unlike Comparative Examples 7 and 8 in which an emulsion was formed, phase separation occurred after passing through the microfluidizer, and thus an emulsion was not formed.

From the formulation implementation test, it was confirmed that the nanoemulsion composition of the present disclosure can improve skin absorption by implementing an emulsion formulation having a small average particle size of 150 to 300 nm.

### Transparency Test

A transparency test was performed on the compositions of the Examples and Comparative Examples.

The transparency test was carried out by applying 2 g of each composition prepared in Example 7 and Comparative Example 9 onto a glass plate to a thickness of 100 µm, and visually observing whether letters placed under the glass plate could be clearly seen through the coated film.

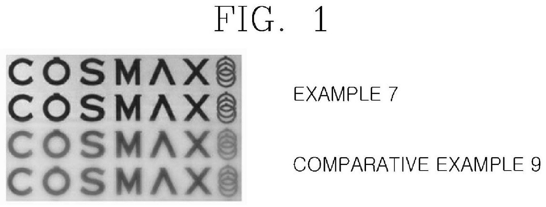

FIG. 1 shows the results of the above test.

As shown in FIG. 1, it was confirmed that Example 7, which is a nanoemulsion composition, was transparent, whereas Comparative Example 9, which is a general emulsion composition, was observed to be opaque.

### Formulation Stability Test

A formulation stability test was performed on the compositions of the Examples and Comparative Examples.

The formulation stability test was carried out by storing each composition prepared in Example 7 and Comparative Example 9 in constant temperature and humidity chambers at 4°C, 25°C, 45°C, and 50°C for 4 weeks, and visually observing changes in the formulation.

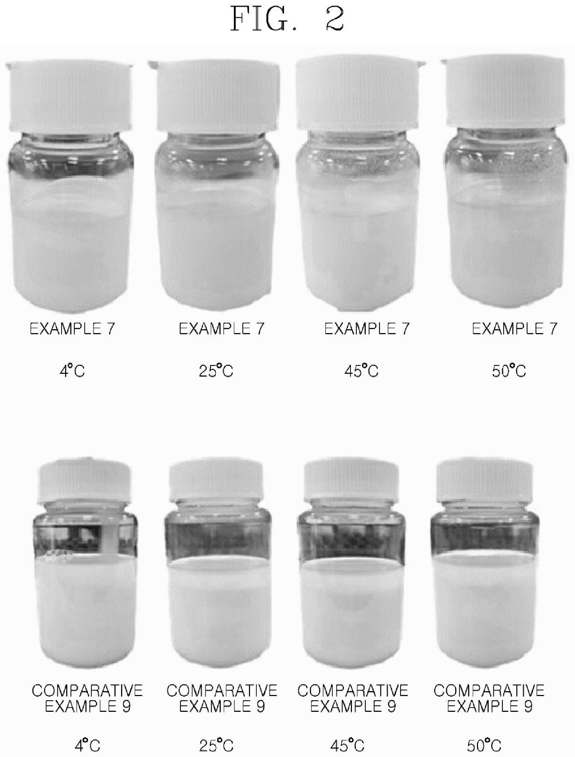

FIG. 2 shows the results of the above test, illustrating changes in the appearance of the compositions.

As shown in FIG. 2, Example 7 maintained formulation stability for 4 weeks at all temperatures, whereas phase separation was observed in Comparative Example 9 at all temperatures.

From the formulation stability test, it was confirmed that the nanoemulsion composition of the present disclosure is stable in formulation, with no phase separation and maintained transparency.

### Moisture Retention Test

A moisture retention test was performed on the compositions of the Examples and Comparative Examples.

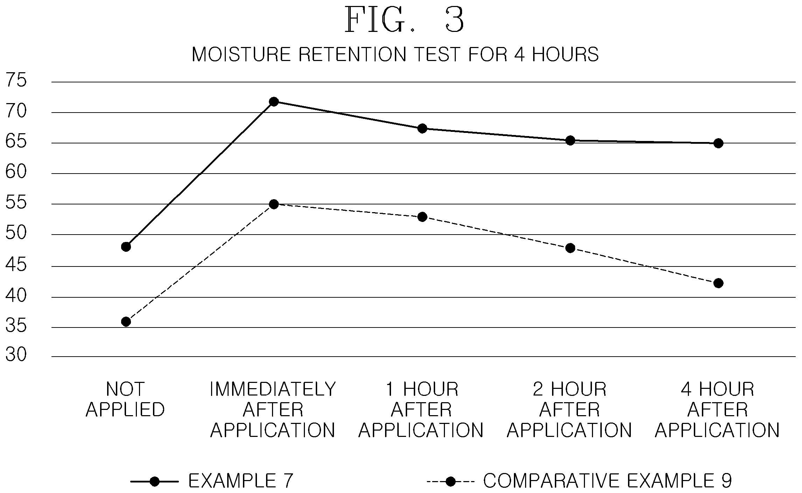

The moisture retention test was carried out by applying the compositions of Example 7 and Comparative Example 9 onto the skin, and measuring skin moisture content immediately after application and after 1 hour, 2 hours, and 4 hours. A corneometer (Corneometer, CK, Germany) was used as the measuring instrument, and the average of three measurements was used and compared with the skin moisture content before application. The results are shown in FIG. 3.

As shown in FIG. 3, it was confirmed that Example 7 not only exhibited higher skin moisture content immediately after application compared to Comparative Example 9, but also maintained the increased skin moisture level for 4 hours.

From the moisture retention test, it was confirmed that the nanoemulsion composition of the present disclosure has excellent moisture retention properties.

### Sensory Evaluation Test

A sensory evaluation test was performed on the compositions of the Examples and Comparative Examples.

The sensory evaluation test was conducted on 20 women aged 20 to 40, who used each composition prepared in Example 7 and Comparative Example 9, and evaluated usability in four categories, including smoothness upon application, absorbability, moisturizing effect, and stickiness. Each category was scored on a scale of 1 to 10, and the average value of the scores from the 20 participants was calculated, and the results are shown in Table 5 below.

### - Evaluation Criteria

8 points or more: very good (⊚), 6-8 points: good (○), 3-6 points: fair (△), less than 3 points: poor (×)

**[TABLE 5]**

| | Ex.7 | C. Ex.9 |
|---|---|---|
| Softness | ○ | ○ |
| Absorbability | ○ | × |
| Moisture retention | ⊚ | △ |
| Stickiness | ⊚ | × |

As shown in Table 5, Example 7 was evaluated as having excellent usability in all categories. In contrast, Comparative Example 9 was evaluated as having poor absorbability and high stickiness.

From the sensory evaluation test, it was confirmed that the nanoemulsion composition of the present disclosure provides a non-sticky and smooth feel, and has excellent absorbability and moisturizing properties.

### <Preparation Example 3 of Nanoemulsion Composition>

Emulsion compositions having different contents of a skin conditioning agent were prepared according to the compositions (wt%) shown in Table 6 below. The specific preparation method of Comparative Examples 12 to 14 is the same as that of Comparative Example 9, and the specific preparation method of Comparative Examples 15 and 16 and Example 8 is the same as that of Example 1.

**[TABLE 6]**

| | Component(wt%) | C. Ex. 12 | C. Ex. 13 | C. Ex. 14 | C. Ex. 15 | C. Ex. 16 | Ex. 8 |
|---|---|---|---|---|---|---|---|
| Oil phase | PEG-10 dimethicone | 10 | 10 | 10 | 10 | 10 | 10 |
| | Dimethicone | 5 | 15 | 25 | 5 | 15 | 25 |
| Water phase | Purified water | Balance | Balance | Balance | Balance | Balance | Balance |
| | Glycerin | 10 | 10 | 10 | 10 | 10 | 10 |
| | Propanediol | 5 | 5 | 5 | 5 | 5 | 5 |
| | Other additives | 5 | 5 | 5 | 5 | 5 | 5 |
| Sum | | 100.0 | 100.0 | 100.0 | 100.0 | 100.0 | 100.0 |

### Formulation Implementation Test

A formulation implementation test was performed on the compositions of the Examples and Comparative Examples.

Each composition prepared in the Examples and Comparative Examples was visually observed, and the particle size of the composition diluted in oil was measured by static light scattering (SLS) using an LA-960 particle size analyzer (Horiba, Japan). The results of the evaluation of formulation appearance and the average particle size are shown in Table 7 below.

### - Criteria for formulation evaluation

⊚: very good, ○: good, △: fair, ×: phase separation

**[TABLE 7]**

| | C. Ex. 12 | C. Ex. 13 | C. Ex. 14 | C. Ex. 15 | C. Ex. 16 | Ex. 8 |
|---|---|---|---|---|---|---|
| Evaluation for formulation implementation | × | ○ | ○ | × | × | ⊚ |
| Avg. diameter(nm) | - | 7210 | 6850 | - | - | 245 |

As shown in Table 7, it was confirmed that Example 8 successfully implemented a nanoemulsion composition having an average particle size of 245 nm. However, in Comparative Examples 15 and 16 having a low content of the skin conditioning agent, phase separation was observed. In addition, phase separation was observed in Comparative Example 12, and Comparative Examples 13 and 14 were confirmed to have large average particle sizes.

From the formulation implementation test, it was confirmed that the nanoemulsion composition of the present disclosure can improve skin absorption by implementing an emulsion formulation having a small average particle size of 150 to 300 nm.

### Formulation Stability Test

A formulation stability test was performed on the compositions of the Examples and Comparative Examples.

The formulation stability test was carried out by storing each composition prepared in Example 8 and Comparative Example 14 in constant temperature and humidity chambers at 4°C, 25°C, 45°C, and 50°C for 4 weeks, and visually observing changes in the formulation.

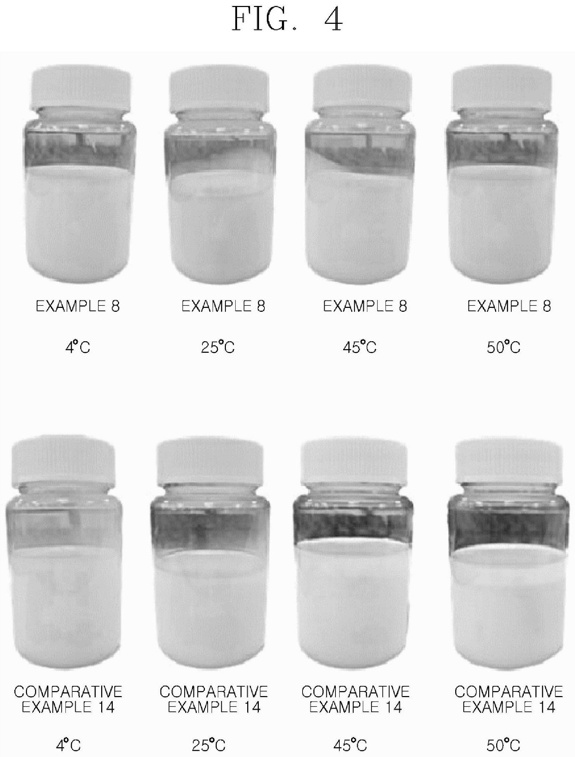

FIG. 4 shows the results of the above test, illustrating changes in the appearance of the compositions.

As shown in FIG. 4, Example 8 maintained formulation stability for 4 weeks at all temperatures. Comparative Example 14 was stable at low temperatures, whereas formulation changes were observed at high temperatures.

From the formulation stability test, it was confirmed that the nanoemulsion composition of the present disclosure is stable in formulation, with no phase separation and maintained transparency.

### <Preparation Example 4 of Nanoemulsion Composition>

Emulsion compositions having different contents of a polyol were prepared according to the compositions (wt%) shown in Table 8 below. The specific preparation method of Comparative Examples 17 to 19 is the same as that of Comparative Example 9, and the specific preparation method of Examples 9 to 11 is the same as that of Example 1.

**[TABLE 8]**

| | Component(wt%) | C. Ex. 17 | C. Ex. 18 | C. Ex. 19 | Ex. 9 | Ex. 10 | Ex. 11 |
|---|---|---|---|---|---|---|---|
| Oil | PEG-10 dimethicone | 10 | 10 | 10 | 10 | 10 | 10 |
| phase | Dimethicone | 20 | 20 | 20 | 20 | 20 | 20 |
| Water phase | Purified water | Balance | Balance | Balance | Balance | Balance | Balance |
| | Glycerin | 10 | 10 | 10 | 10 | 10 | 10 |
| | Propanediol | 10 | 20 | 40 | 10 | 20 | 40 |
| | Other additives | 5 | 5 | 5 | 5 | 5 | 5 |
| Sum | | 100.0 | 100.0 | 100.0 | 100.0 | 100.0 | 100.0 |

### Formulation Implementation and Stability Test

A formulation implementation and stability test was performed on the compositions of the Examples and Comparative Examples.

Each composition prepared in Examples 9 to 11 and Comparative Examples 17 to 19 was visually observed to confirm formulation implementation, and formulation stability was evaluated by storing the compositions of Example 9 and Comparative Example 17 in constant temperature and humidity chambers at 4°C, 25°C, 45°C, and 50°C for 4 weeks and visually observing changes in the formulation.

As a result of the formulation implementation test, favorable formulations were observed in Examples 9 to 11 and Comparative Examples 17 to 19, and in particular, it was confirmed that Examples 9 and 10 exhibited very good formulations.

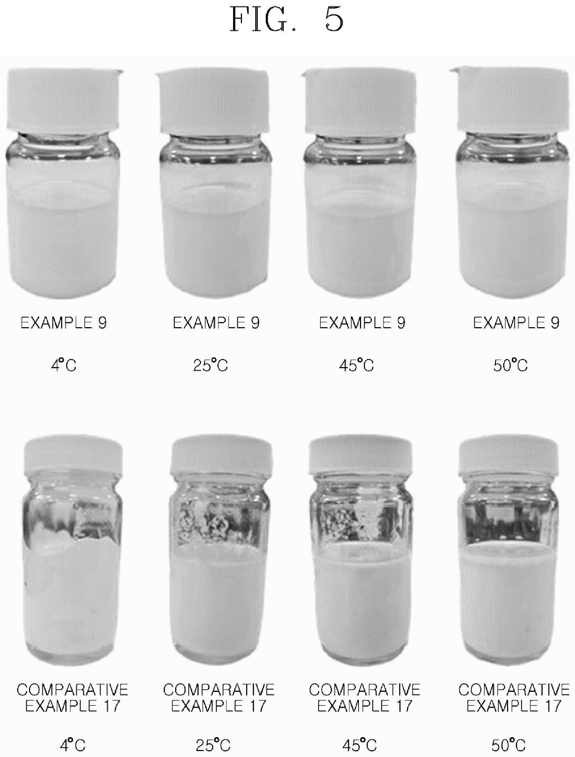

FIG. 5 shows the results of the formulation stability test, illustrating changes in the appearance of the compositions.

As shown in FIG. 5, Example 9 maintained formulation stability for 4 weeks at all temperatures. Comparative Example 17 was stable at low temperatures, whereas formulation changes were observed at high temperatures.

From the formulation stability test, it was confirmed that the nanoemulsion composition of the present disclosure is stable in formulation, with no phase separation and maintained transparency.

### Sensory Evaluation Test

A sensory evaluation test was performed on the compositions of the Examples and Comparative Examples.

The sensory evaluation test was conducted on 20 women aged 20 to 40, who used each composition prepared in Examples 9 to 11 and Comparative Examples 17 to 19, and evaluated usability in three categories, including absorbability, moisturizing effect, and stickiness upon application. Each category was scored on a scale of 1 to 10, and the average value of the scores from the 20 participants was calculated, and the results are shown in Table 9 below.

### - Evaluation Criteria

8 points or more: very good (⊚), 6-8 points: good (○), 3-6 points: fair (△), less than 3 points: poor (×)

**[TABLE 9]**

| | C. Ex. 17 | C. Ex. 18 | C. Ex. 19 | Ex. 9 | Ex. 10 | Ex. 11 |
|---|---|---|---|---|---|---|
| Absorbability | ○ | × | × | ⊚ | ○ | △ |
| Moisture retention | △ | ○ | ○ | ○ | ⊚ | ⊚ |
| Stickiness | ○ | × | × | ⊚ | ○ | △ |

As shown in Table 9, Examples 9 to 11 were evaluated as having excellent usability in all categories. In contrast, Comparative Examples 18 and 19 were evaluated as having poor absorbability and high stickiness. Comparative Example 17 received lower evaluations than the Examples, but was generally evaluated as good overall.

From the sensory evaluation test, it was confirmed that the nanoemulsion composition of the present disclosure provides a non-sticky and smooth feel, and has excellent absorbability and moisturizing properties.

### <Preparation Example 5 of Nanoemulsion Composition>

Nanoemulsion compositions comprising various silicone-based surfactants were prepared according to the compositions (wt%) shown in Table 10 below, and a formulation implementation test was performed. The specific preparation method and test method are the same as those of Preparation Example 1.

**[TABLE 10]**

| | Component(wt%) | Ex. 7 | Ex. 12 | Ex. 13 | Ex. 14 | Ex. 15 | Ex. 16 |
|---|---|---|---|---|---|---|---|
| Oil phase | PEG-10 dimethicone | 10 | - | - | - | - | - |
| | Cetyl PEG/PPG-10/1 Dimethicone | - | 10 | - | - | - | - |
| | Lauryl PEG -9 polydimethylsiloxyethyl dimethicone | - | - | 10 | - | - | - |
| | Polyglyceryl-3 polydimethylsiloxyethyl dimethicone | - | - | - | 10 | - | - |
| | Lauryl polyglyceryl-3 polydimethylsiloxyethyl dimethicone | - | - | - | - | 10 | - |
| | PEG/PPG-18/18 dimethicone | - | - | - | - | - | 10 |
| | Dimethicone | 20 | 20 | 20 | 20 | 20 | 20 |
| Water phase | Purified water | Balance | Balance | Balance | Balance | Balance | Balance |
| | Glycerin | 10 | 10 | 10 | 10 | 10 | 10 |
| | Propanediol | 5 | 5 | 5 | 5 | 5 | 5 |
| | Other additives | 5 | 5 | 5 | 5 | 5 | 5 |
| Sum | | 100.0 | 100.0 | 100.0 | 100.0 | 100.0 | 100.0 |

The results of the evaluation of formulation appearance and the average particle size are shown in Table 11 below.

**[TABLE 11]**

| | Ex. 7 | Ex. 12 | Ex. 13 | Ex. 14 | Ex. 15 | Ex. 16 |
|---|---|---|---|---|---|---|
| Evaluation for formulation implementation | ⊚ | ⊚ | ⊚ | ⊚ | ⊚ | ⊚ |
| Avg. diameter(nm) | 260 | 350 | 220 | 500 | 480 | 400 |

As shown in Table 11, Examples 7 and 12 to 16, which include silicone-based surfactants, successfully implemented nanoemulsion compositions having an average particle size of 220 to 500 nm.

### <Preparation Example 6 of Nanoemulsion Composition>

Nanoemulsion compositions comprising various fatty acid ester-based surfactants were prepared according to the compositions (wt%) shown in Table 12 below, and a formulation implementation test was performed. The specific preparation method and test method are the same as those of Preparation Example 1.

**[TABLE 12]**

| | Component(wt%) | Ex. 17 | Ex. 18 | Ex. 19 | Ex. 20 | Ex. 21 | Ex. 22 |
|---|---|---|---|---|---|---|---|
| Oil phase | Polyglyceryl-2 dipolyhydroxystearate | 10 | - | - | - | - | - |
| | Polyglyceryl-2 diisostearate | - | 10 | - | - | - | - |
| | Polyglyceryl-3 polyricinoleate | - | - | 10- | - | - | - |
| | Polyglyceryl-3 diisostearate | - | - | - | 10 | - | - |
| | Polyglyceryl-4 isostearate | - | - | - | - | 10 | - |
| | Polyglyceryl-10 decaisostearate | - | - | - | - | - | 10 |
| | Caprylic /capric triglyceride | 20 | 20 | 20 | 20 | 20 | 20 |
| Water phase | Purified water | Balance | Balance | Balance | Balance | Balance | Balance |
| | Glycerin | 10 | 10 | 10 | 10 | 10 | 10 |
| | Propanediol | 5 | 5 | 5 | 5 | 5 | 5 |
| | Other additives | 5 | 5 | 5 | 5 | 5 | 5 |
| Sum | | 100.0 | 100.0 | 100.0 | 100.0 | 100.0 | 100.0 |

The results of the evaluation of formulation appearance and the average particle size are shown in Table 13 below.

**[TABLE 13]**

| | Ex. 17 | Ex. 18 | Ex. 19 | Ex. 20 | Ex. 21 | Ex. 22 |
|---|---|---|---|---|---|---|
| Evaluation for formulation implementation | ⊚ | ⊚ | ⊚ | ⊚ | ⊚ | ⊚ |
| Avg. diameter(nm) | 210 | 210 | 220 | 220 | 230 | 250 |

As shown in Table 13, Examples 17 to 22, which include fatty acid ester-based surfactants, successfully implemented nanoemulsion compositions having an average particle size of 210 to 250 nm.

### <Preparation Example 7 of Nanoemulsion Composition>

Nanoemulsion compositions having different types of skin conditioning agents were prepared according to the compositions (wt%) shown in Table 14 below, and a formulation implementation test was performed. The specific preparation method and test method are the same as those of Preparation Example 1.

**[TABLE 14]**

| | Component(wt%) | Ex. 7 | Ex. 23 | Ex. 24 | Ex. 25 | Ex. 26 | Ex. 27 |
|---|---|---|---|---|---|---|---|
| Oil phase | PEG-10 dimethicone | 10 | 10 | 10 | 10 | 10 | 10 |
| | Dimethicone 2cs | 20 | - | - | - | - | - |
| | Dimethicone 6cs | - | 20 | - | - | - | - |
| | Cyclopentasiloxane | - | - | 20 | - | - | - |
| | Methyl trimethicone | - | - | - | 20 | - | - |
| | Caprylyl methicone | - | - | - | - | 20 | - |
| | Phenyl trimethicone | - | - | - | - | - | 20 |
| Water phase | Purified water | Balance | Balance | Balance | Balance | Balance | Balance |
| | Glycerin | 10 | 10 | 10 | 10 | 10 | 10 |
| | Propanediol | 5 | 5 | 5 | 5 | 5 | 5 |
| | Other additives | 5 | 5 | 5 | 5 | 5 | 5 |
| Sum | | 100.0 | 100.0 | 100.0 | 100.0 | 100.0 | 100.0 |

The results of the evaluation of formulation appearance and the average particle size are shown in Table 15 below.

**[TABLE 15]**

| | Ex. 7 | Ex. 23 | Ex. 24 | Ex. 25 | Ex. 26 | Ex. 27 |
|---|---|---|---|---|---|---|
| Evaluation for formulation implementation | ⊚ | ⊚ | ⊚ | ⊚ | ⊚ | ⊚ |
| Avg. diameter(nm) | 260 | 270 | 250 | 250 | 260 | 290 |

As shown in Table 15, Examples 7 and 23 to 27, which include silicone-based oils, successfully implemented nanoemulsion compositions having an average particle size of 250 to 290 nm.

### <Preparation Example 8 of Nanoemulsion Composition>

Nanoemulsion compositions having different types of skin conditioning agents were prepared according to the compositions (wt%) shown in Table 16 below, and a formulation implementation test was performed. The specific preparation method and test method are the same as those of Preparation Example 1.

**[TABLE 16]**

| | Component(wt%) | Ex. 21 | Ex. 28 | Ex. 29 | Ex. 30 | Ex. 31 | Ex. 32 |
|---|---|---|---|---|---|---|---|
| Oil phase | Polyglyceryl-4 isostearate | 10 | 10 | 10 | 10 | 10 | 10 |
| | Caprylic/capric triglyceride | 20 | - | - | - | - | - |
| | Cetylethylhexanoate | - | 20 | - | - | - | - |
| | Isononyl isonanoate | - | - | 20 | - | - | - |
| | Squalane | - | - | - | 20 | - | - |
| | C15-19 alkane | - | - | - | - | 20 | - |
| | Hydrogenated coconut oil | - | - | - | - | - | 20 |
| Water phase | Purified water | Balance | Balance | Balance | Balance | Balance | Balance |
| | Glycerin | 10 | 10 | 10 | 10 | 10 | 10 |
| | Propanediol | 5 | 5 | 5 | 5 | 5 | 5 |
| | Other additives | 5 | 5 | 5 | 5 | 5 | 5 |
| Sum | | 100.0 | 100.0 | 100.0 | 100.0 | 100.0 | 100.0 |

The results of the evaluation of formulation appearance and the average particle size are shown in Table 17 below.

**[TABLE 17]**

| | Ex. 21 | Ex. 28 | Ex. 29 | Ex. 30 | Ex. 31 | Ex. 32 |
|---|---|---|---|---|---|---|
| Evaluation for formulation implementation | ⊚ | ⊚ | ⊚ | ⊚ | ⊚ | ⊚ |
| Avg. diameter(nm) | 230 | 230 | 230 | 220 | 220 | 220 |

As shown in Table 17, Examples 21 and 28 to 32, which include ester-based oils or hydrocarbon-based oils, successfully implemented nanoemulsion compositions having an average particle size of 220 to 230 nm.

### <Preparation Example 9 of Nanoemulsion Composition>

Nanoemulsion compositions having different types of polyols were prepared according to the compositions (wt%) shown in Table 18 below, and a formulation implementation test was performed. The specific preparation method and test method are the same as those of Preparation Example 1.

**[TABLE 18]**

| | Component(wt%) | Ex. 33 | Ex. 34 | Ex. 35 | Ex. 36 | Ex. 37 | Ex. 38 |
|---|---|---|---|---|---|---|---|
| Oil phase | PEG-10 dimethicone | 10 | 7 | 10 | 5 | 7 | 10 |
| | Dimethicone | 20 | 20 | 20 | 20 | 20 | 20 |
| Water phase | Purified water | Balance | Balance | Balance | Balance | Balance | Balance |
| | Glycerin | 15 | - | - | - | - | - |
| | Propanediol | - | 15 | - | - | - | - |
| | Butylene glycol | - | - | 15 | - | - | - |
| | Dipropylene glycol | - | - | - | 15 | - | - |
| | Diglycerin | - | - | - | - | 15 | - |
| | Methylpropane diol | - | - | - | - | - | 15 |
| | Other additives | 5 | 5 | 5 | 5 | 5 | 5 |
| Sum | | 100.0 | 100.0 | 100.0 | 100.0 | 100.0 | 100.0 |

The results of the evaluation of formulation appearance and the average particle size are shown in Table 19 below.

**[TABLE 19]**

| | Ex. 33 | Ex. 34 | Ex. 35 | Ex. 36 | Ex. 37 | Ex. 38 |
|---|---|---|---|---|---|---|
| Evaluation for formulation implementation | ⊚ | ⊚ | ⊚ | ⊚ | ⊚ | ⊚ |
| Avg. diameter(nm) | 260 | 250 | 250 | 260 | 270 | 250 |

As shown in Table 19, it was confirmed that in Examples 33 to 38 including different polyols, nanoemulsion compositions having an average particle size of 250 to 270 nm were successfully implemented.

### <Preparation Example 10 of Nanoemulsion Composition>

Nanoemulsion compositions having the same components were prepared under various pressures according to the compositions (wt%) shown in Table 20 below.

**[TABLE 20]**

| | Component(wt%) | Ex. 39 | Ex. 40 | Ex. 41 | Ex. 42 | C. Ex. 20 | C. Ex. 21 |
|---|---|---|---|---|---|---|---|
| Oil phase | PEG-10 dimethicone | 10 | 10 | 10 | 10 | 10 | 10 |
| | Dimethicone | 20 | 20 | 20 | 20 | 20 | 20 |
| Water phase | Purified water | Balance | Balance | Balance | Balance | Balance | Balance |
| | Glycerin | 10 | 10 | 10 | 10 | 10 | 10 |
| | Propanediol | 5 | 5 | 5 | 5 | 5 | 5 |
| | Other additives | 5 | 5 | 5 | 5 | 5 | 5 |
| Sum | | 100.0 | 100.0 | 100.0 | 100.0 | 100.0 | 100.0 |
| Pressure | | 100bar | 500bar | 1000bar | 1500bar | 1700bar | 2000bar |

Specifically, after weighing according to the composition of Table 20, an oil phase part and an aqueous phase part were each heated and dissolved at 30°C, and the aqueous phase part was gradually added to the oil phase part while stirring at 3000 rpm for 10 minutes using a homogenizer to prepare a conventional emulsion. The conventional emulsion was then subjected to an emulsification process twice using a microfluidizer at a temperature of 30°C and a pressure of 100 to 2000 bar to prepare water-in-oil nanoemulsion compositions of Examples 39, 40, 41, and 42 and Comparative Examples 20 and 21.

### Formulation Implementation Test

A formulation implementation test was performed on the compositions of the Examples and Comparative Examples.

Each composition prepared in the Examples and Comparative Examples was visually observed, and the results of the evaluation of formulation appearance are shown in Table 21 below.

### - Criteria for formulation evaluation

⊚: very good, ○: good, △: fair, ×: phase separation

**[TABLE 21]**

| | Ex. 39 | Ex. 40 | Ex. 41 | Ex. 42 | C. Ex. 20 | C. Ex. 21 |
|---|---|---|---|---|---|---|
| Evaluation for formulation implementation | ⊚ | ⊚ | ⊚ | ○ | × | × |

As shown in Table 21, it was confirmed that in Examples 39, 40, 41, and 42, nanoemulsion compositions were successfully implemented. However, in Comparative Examples 20 and 21, phase separation occurred after passing through the microfluidizer, and thus an emulsion was not formed.

### <Preparation Example of Cosmetic Composition>

Cosmetic compositions were prepared according to the compositions (wt%) shown in Table 22 below.

**[TABLE 22]**

| | Component(wt%) | Ex. 43 | Ex. 44 | Ex. 45 | C. Ex. 22 | C. Ex. 23 | C. Ex. 24 |
|---|---|---|---|---|---|---|---|
| 1 | Nanoemulsion composition of Ex. 7 | 50 | 50 | 50 | - | - | - |
| 2 | General emulsion composition of C. Ex. 9 | - | - | - | 50 | 50 | 50 |
| 3 | Titanium oxide | 10 | 20 | 20 | 10 | 10 | 20 |
| 4 | Iron oxide yellow | 2 | 2 | 2 | 2 | 2 | 2 |
| 5 | Iron oxide red | 0.5 | 0.5 | 0.5 | 0.5 | 0.5 | 0.5 |
| 6 | Iron oxide black | 0.1 | 0.1 | 0.1 | 0.1 | 0.1 | 0.1 |
| 7 | Dimethicone | 10 | 10 | 10 | 10 | 10 | 10 |
| 8 | Thickener | 0.1 | 0.3 | 0.5 | 0.2 | 0.6 | 1.0 |
| 9 | Purified water | Balance | Balance | Balance | Balance | Balance | Balance |
| Sum | | 100.0 | 100.0 | 100.0 | 100.0 | 100.0 | 100.0 |

Specifically, after adding a powder to the nanoemulsion composition of Example 7, the mixture was stirred at 3000 rpm for 20 minutes using a homogenizer to prepare the cosmetic compositions of Examples 43, 44, and 45. Comparative Examples 22, 23, and 24 were prepared in the same manner as Example 43, except that a powder was added to the general emulsion composition of Comparative Example 9.

### Viscosity Measurement

The viscosity of the cosmetic compositions of the Examples and Comparative Examples was measured.

The viscosity was measured using a Brookfield viscometer under conditions of 4 spindle, 12 rpm, 1 minute, and 25°C, and the results are shown in Table 23 below.

**[TABLE 23]**

| | Ex. 43 | Ex. 44 | Ex. 45 | C. Ex. 22 | C. Ex. 23 | C. Ex. 24 |
|---|---|---|---|---|---|---|
| Viscosity(cps) | 1,000 | 5,000 | 10,000 | 1,000 | 5,000 | 10,000 |

As shown in Table 23, Example 43 and Comparative Example 22, Example 44 and Comparative Example 23, and Example 45 and Comparative Example 24 were found to have similar levels of viscosity.

### Formulation Stability Test

A formulation stability test was performed on the cosmetic compositions of the Examples and Comparative Examples.

The formulation stability test was carried out by storing each cosmetic composition prepared in Examples 43 to 45 and Comparative Examples 22 to 24 in constant temperature and humidity chambers at 4°C, 25°C, 45°C, and 50°C for 4 weeks, and visually observing changes in the formulation.

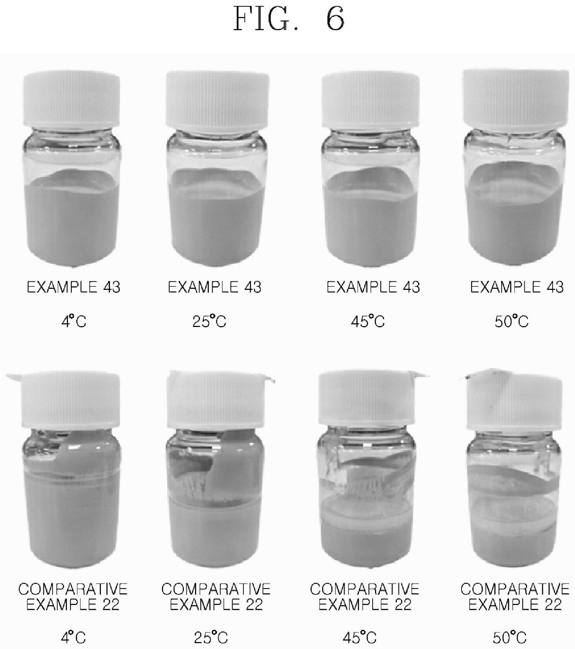

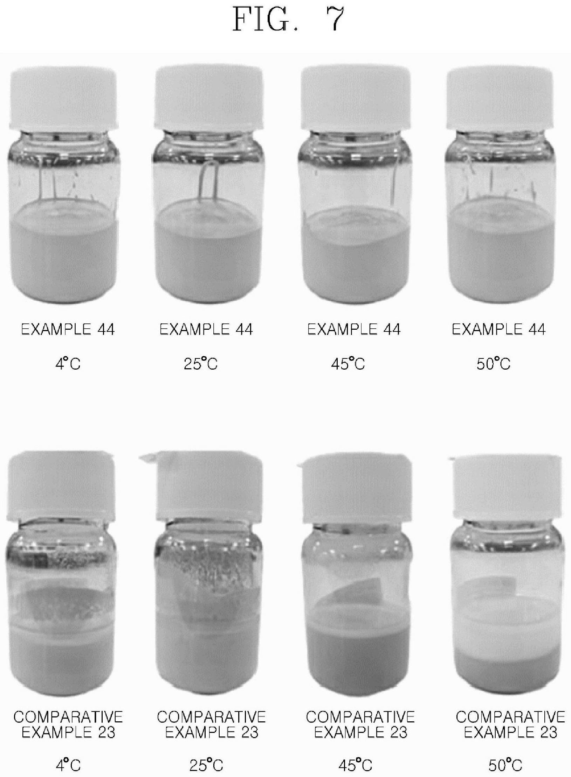

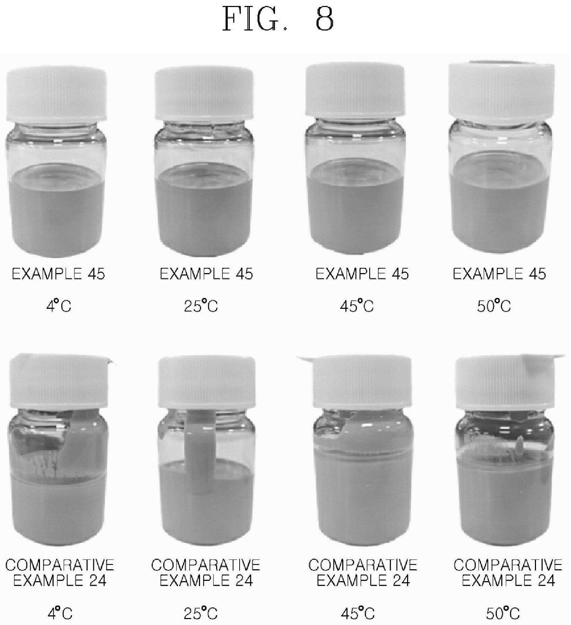

FIGS. 6 to 8 show the results of the above test, illustrating changes in the appearance of the compositions.

As shown in FIGS. 6 to 8, Examples 43 to 45 maintained formulation stability for 4 weeks at all temperatures. In contrast, in Comparative Examples 22 to 24, pigments were separated even at low temperatures to form bands, and oil separation was also observed, and more severe formulation changes were observed at high temperatures.

From the formulation stability test, it was confirmed that the nanoemulsion cosmetic composition of the present disclosure is stable in formulation without oil separation even at a low viscosity of 10,000 cps or less.

### [Industrial Applicability]

The present disclosure can be applied to cosmetics.

## Claims

1. A method for preparing a water-in-oil nanoemulsion composition, the method comprising the steps of:
preparing an oil phase comprising a surfactant and a skin conditioning agent (S1);
preparing an aqueous phase comprising a polyol (S2);
mixing the oil phase and the aqueous phase (S3); and
emulsifying the mixture of step (S3) using a microfluidizer (S4).

2. The method of claim 1, wherein the surfactant comprises one or more selected from the group consisting of PEG-10 dimethicone, cetyl PEG/PPG-10/1 dimethicone, lauryl PEG-9 polydimethylsiloxyethyl dimethicone, lauryl PEG-10 tris(trimethylsiloxy)silyl ethyl dimethicone, PEG-9 polydimethylsiloxyethyl dimethicone, bis-PEG/PPG-14/14 dimethicone, bis-PEG/PPG-20/20 dimethicone, PEG/PPG-18/18 dimethicone, PEG/PPG-19/19 dimethicone, PEG/PPG-20/20 dimethicone, PEG-8 dimethicone, bis-PEG-12 dimethicone, lauryl PEG-8 dimethicone, polyglyceryl-3 polydimethylsiloxyethyl dimethicone, lauryl polyglyceryl-3 polydimethylsiloxyethyl dimethicone, dimethicone/PEG-10/15 crosspolymer, dimethicone/polyglycerin-3 crosspolymer, lauryl dimethicone/polyglycerin-3 crosspolymer, sorbitan stearate, sorbitan oleate, sorbitan isostearate, sorbitan sesquioleate, PEG-30 dipolyhydroxystearate, polyglyceryl-2 isostearate, polyglyceryl-2 diisostearate, polyglyceryl-2 tetraisostearate, polyglyceryl-2 oleate, polyglyceryl-2 dipolyhydroxystearate, polyglyceryl-3 distearate, polyglyceryl-3 diisostearate, polyglyceryl-3 polyricinoleate, polyglyceryl-4 isostearate, polyglyceryl-4 oleate, polyglyceryl-4 tristearate, polyglyceryl-4 pentastearate, polyglyceryl-4 pentaoleate, polyglyceryl-4 polyricinoleate, polyglyceryl-4 diisostearate/polyhydroxystearate/sebacate, polyhydroxystearic acid, polyglyceryl-6 pentastearate, polyglyceryl-6 pentaoleate, polyglyceryl-6 polyricinoleate, polyglyceryl-10 decaethylhexanoate, polyglyceryl-10 decastearate, polyglyceryl-10 decaisostearate, polyglyceryl-10 pentaisostearate, polyglyceryl-10 decaoleate, polyglyceryl-10 decaoleate, and polyglyceryl-10 dodecabehenate.

3. The method of claim 1,
wherein the surfactant is contained in an amount of 10 to 20 wt% based on the total weight of the composition.

4. The method of claim 1,
wherein the skin conditioning agent comprises one or more selected from the group consisting of hydrocarbon oils, ester oils, silicone oils, polyesters, fatty acids and/or fatty alcohols, animal oils, vegetable oils, mineral oils, and mixtures thereof.

5. The method of claim 1,
wherein the skin conditioning agent comprises one or more selected from the group consisting of disiloxane, trisiloxane, dimethicone, methyl trimethicone, cyclopentasiloxane, cyclohexasiloxane, caprylyl methicone, cetyl dimethicone, phenyl trimethicone, diphenylsiloxy phenyl trimethicone, diphenyl dimethicone, trimethylsiloxy phenyl dimethicone, trimethylsiloxysilicate, phenylpropyl dimethylsiloxysilicate, acrylates/dimethicone copolymer, acrylates/polytrimethylsiloxymethacrylate copolymer, acrylates/stearyl acrylate/dimethicone methacrylate copolymer, polypropylsilsesquioxane, polymethylsilsesquioxane, polyphenylsilsesquioxane, vinyl dimethicone/methicone silsesquioxane crosspolymer, dimethicone/vinyl dimethicone crosspolymer, dimethicone crosspolymer, dimethicone/PEG-10/15 crosspolymer, dimethicone/polyglycerin-3 crosspolymer, lauryl dimethicone/polyglycerin-3 crosspolymer, dilinoleic acid/butanediol copolymer, castor oil/IPDI copolymer, coco-caprylate/caprate, triheptanoin, dipropylene glycol dibenzoate, octyldodecanol, tridecyl trimellitate, caprylic/capric triglyceride, decyl cocoate, C12-15 alkyl benzoate, isononyl isononanoate, cetyl ethylhexanoate, butylene glycol dicaprylate/dicaprate, isododecane, C15-19 alkane, C9-12 alkane, squalane, and hydrogenated coconut oil.

6. The method of claim 1,
wherein the skin conditioning agent is contained in an amount of 20 to 50 wt% based on the total weight of the composition.

7. The method of claim 1,
wherein the surfactant and the skin conditioning agent is contained in a sum amount of 30 to 70 wt% based on the total weight of the composition.

8. The method of claim 1,
wherein a weight ratio of the surfactant to the skin conditioning agent is 1:1 to 1:5.

9. The method of claim 1,
wherein the polyol comprises one or more selected from the group consisting of propanediol, propylene glycol, methylpropanediol, dipropylene glycol, butylene glycol, diglycerin, glycerin, pentylene glycol, and 1,2-hexanediol.

10. The method of claim 1,
wherein the polyol is contained in an amount of 0.1 to 50 wt% based on the total weight of the composition.

11. The method of claim 1,
wherein step (S4) is performed under a pressure of 100 to 1500 bar.

12. The method of claim 1,
wherein step (S4) is performed at a temperature of 0 to 70°C.

13. The method of claim 1,
wherein step (S4) is performed one or more times.

14. A water-in-oil nanoemulsion composition, comprising a surfactant, a skin conditioning agent, and a polyol.

15. A water-in-oil sunscreen and makeup nanoemulsion cosmetic composition, comprising the water-in-oil nanoemulsion composition of claim 14.

16. The water-in-oil sunscreen and makeup nanoemulsion cosmetic composition of claim 15,
wherein the water-in-oil nanoemulsion composition is contained in an amount of 1 to 99 wt% based on the total weight of the water-in-oil sunscreen and makeup nanoemulsion cosmetic composition.

17. The water-in-oil sunscreen and makeup nanoemulsion cosmetic composition of claim 15, further comprising a powder.

18. The water-in-oil sunscreen and makeup nanoemulsion cosmetic composition of claim 17,
wherein the powder comprises one or more selected from the group consisting of titanium dioxide, fine titanium dioxide, zinc oxide, yellow iron oxide, red iron oxide, black iron oxide, ultramarine, and chromium oxide green.

19. The water-in-oil sunscreen and makeup nanoemulsion cosmetic composition of claim 17,
wherein the powder is contained in an amount of 0.1 to 50 wt% based on the total weight of the water-in-oil sunscreen and makeup nanoemulsion cosmetic composition.
